# EUROPEAN PATENT APPLICATION

(11) **EP 4 817 596 A1**
(43) Date of publication of application: **07.10.2026**
(21) Application number: 26170742.6
(22) Date of filing: 03.04.2026
(51) Int. Cl.: A61K 8/73, A61Q 5/02, A61Q 19/10

(54) **COSMETIC COMPOSITIONS INCORPORATING INULIN AS A THICKENING AGENT**

(30) Priority: 03.04.2025 NL 2040110
(71) Applicant: Gobiotics, 4631SL Hoogerheide (NL)
(72) Inventor: Gonry, Patrick, 2910 Essen (BE); Piotrowska, Anna, 2170 Merksem (BE)
(74) Representative: De Clercq & Partners

(57) **Abstract**

The present invention relates to cosmetic compositions comprising at least 10% by weight of inulin, based on the combined weight of inulin and water, and a surfactant selected from the group consisting of anionic surfactants with a Hydrophilic-Lipophilic Balance (HLB) of at least 20, non-ionic surfactants, and mixtures thereof, and optionally further comprising an amphoteric surfactant. These compositions may also include additional customary components such as water, preservatives, stabilizers, chelating agents, hydrolyzed proteins, emulsifiers, and oils, which contribute to the overall efficacy, stability, and sensory attributes of the product. Intended primarily for rinse-off applications, the compositions can be utilized among others as hair care, scalp care, or beard care treatments, as well as in products like shampoos, body washes, shower gels, shower butter, soap, makeup removers, facial cleansers, scrubs, or toothpaste.

## Description

### FIELD

The present invention relates to cosmetic compositions, particularly those formulated for rinse-off applications. These compositions incorporate natural ingredients to enhance product performance and consumer experience.

### BACKGROUND

The cosmetic industry is currently experiencing a significant shift towards the use of natural and ecological ingredients, driven by an increased emphasis on sustainability and consumer demand for more environmentally friendly products. Traditionally, cosmetics have relied heavily on petrochemically sourced and synthetic ingredients, which have provided reliable performance and stability. However, as the industry moves away from these traditional formulations, cosmetic formulators face the challenge of finding natural replacements that can match the effectiveness of their synthetic counterparts.

A critical aspect of this transition is the formulation of products that contain surfactants, such as shampoos, shower gels, shower butters, soaps, makeup removers, scrubs, and toothpastes. The limited availability of natural thickening agents presents a formidable challenge, as they must be compatible with a variety of surfactants, achieve a smooth texture, provide good viscosity, ensure stability, and maintain an appealing aspect.

Currently, there is no single natural thickener that can meet these requirements. The formulation of a natural surfactant-containing product often involves a compromise between these various factors, as natural thickeners may not perform consistently across different formulations. This ongoing struggle underscores the need for innovative solutions that can bridge the gap between consumer expectations and the industry's shift towards natural formulations.

EP 1 371 353 B1 discloses the use of inulin as a thickening agent in combination with cationic surfactants for cosmetic compositions.

### SUMMARY

The present invention is at least in part based on the inventors' discovery that inulin, when used in specific concentrations and combined with an anionic surfactant possessing a Hydrophilic-Lipophilic Balance (HLB) of at least 20 and/or a non-ionic surfactant can effectively serve as a natural thickening agent in cosmetic compositions. The inventors discovered that inulin can achieve the desired viscosity, stability, and texture in such formulations that include anionic, and/or non-ionic surfactants. This innovative approach addresses the limitations of existing natural thickeners, providing an effective solution for formulating high-performance, natural rinse-off products.

In an aspect, the invention provides a cosmetic composition comprising:
at least 10% by weight of inulin based on combined weight of inulin and water in the composition, and
one or more surfactant selected from the group consisting of an anionic surfactant with Hydrophilic-Lipophilic Balance (HLB) at least 20, a non-ionic surfactant, and mixtures thereof.

In a further aspect, the invention provides a method for thickening a cosmetic composition comprising one or more surfactant selected from the group consisting of an anionic surfactant with HLB at least 20, a non-ionic surfactant, and mixtures thereof, the method comprising including in the composition at least 10% by weight of inulin based on the combined weight of inulin and water in the composition.

A further aspect provides use of inulin in an amount of at least 10% by weight based on the combined weight of inulin and water in a cosmetic composition for thickening the cosmetic composition which comprises one or more surfactant selected from the group consisting of an anionic surfactant with HLB at least 20, a non-ionic surfactant, and mixtures thereof.

These and further aspects and preferred embodiments of the invention are described in the following sections and in the appended claims. The subject-matter of the appended claims is hereby specifically incorporated in this specification.

### DESCRIPTION OF EMBODIMENTS

As used herein, the singular forms "a", "an", and "the" include both singular and plural referents unless the context clearly dictates otherwise.

The terms "comprising", "comprises" and "comprised of" as used herein are synonymous with "including", "includes", "containing", or "contains", and are inclusive or open-ended and do not exclude additional, non-recited members, elements or method steps. The terms also encompass "constituted of", "consists in", "consisting of", and "consists of", and also the terms "consisting essentially of", "consisting essentially in" and "consists essentially of", which enjoy well-established meanings in patent terminology.

The recitation of numerical ranges by endpoints includes all intervening values between the lower and upper endpoints, as well as the recited endpoints. Intervening values may be integers or, where applicable, fractions, i.e., more broadly any real numbers such as any rational numbers. This applies to numerical ranges irrespective of whether they are introduced by the expression "from... to..." or the expression "between... and..." or another expression. Any numerical range recited herein is intended to include all sub-ranges subsumed therein. For example, each sub-range between any stated value in a stated range and any other stated value in that stated range is also specifically disclosed. Each sub-range between any stated value in a stated range and either the lower endpoint or the upper endpoint of the stated range is also specifically disclosed. The stated value may be an isolated value or an endpoint of a range subsumed by or overlapping with the stated range. For example, for a stated range with lower endpoint L1 and upper endpoint U1 (i.e., stated range L1-U1) and a stated sub-range nested within the stated range with lower endpoint L2 and upper endpoint U2 (i.e., stated sub-range L2-U2), also specifically disclosed are the subranges L1-L2, L1-U2, L2-U1, and U2-U1.

The terms "about" or "approximately" as used herein when referring to a measurable value such as a parameter, an amount, a temporal duration, and the like, are meant to encompass variations of and from the specified value, such as variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

Whereas the terms "one or more" or "at least one", such as one or more members or at least one member of a group of members, is clear *per se,* by means of further exemplification, the term encompasses *inter alia* a reference to any one of said members, or to any two or more of said members, such as, e.g., any ≥3, ≥4, ≥5, ≥6 or ≥7 etc. of said members, and up to all said members. In another example, "one or more" or "at least one" may refer to 1, 2, 3, 4, 5, 6, 7 or more.

As used herein, the term "and/or" when used in a list of two or more items, means that any one of the listed items can be employed by itself or any combination of two or more of the listed items can be employed. For example, if a list is described as comprising group A, B, and/or C, the list can comprise A alone, B alone, C alone, A and B in combination, A and C in combination, B and C in combination, or A, B, and C in combination.

The discussion of the background to the invention herein is included to explain the context of the invention. This is not to be taken as an admission that any of the material referred to was published, known, or part of the common general knowledge in any country as of the priority date of any of the claims.

Throughout this disclosure, various publications, patents and published patent specifications are referenced by an identifying citation. All documents cited in the present specification are hereby incorporated by reference in their entirety. In particular, the teachings or sections of such documents herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the invention. When specific terms are defined in connection with a particular aspect of the invention or a particular embodiment of the invention, such connotation or meaning is meant to apply throughout this specification, i.e., also in the context of other aspects or embodiments of the invention, unless otherwise defined.

In the following passages, different aspects or embodiments of the invention are defined in more detail. Each aspect or embodiment so defined may be combined with any other aspect(s) or embodiment(s) unless clearly indicated to the contrary. In particular, any feature indicated as being preferred or advantageous may be combined with any other feature or features indicated as being preferred or advantageous.

Reference throughout this specification to "one embodiment", "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

Similarly, it should be appreciated that in the description of illustrative embodiments of the invention, various features of the invention are sometimes grouped together in a single embodiment, figure, or description thereof for the purpose of streamlining the disclosure and aiding in the understanding of one or more of the various inventive aspects.

As corroborated by the experimental section, which illustrates certain representative embodiments of the present invention, the inventors provide improved cosmetic formulations that include anionic surfactants, and/or non-ionic surfactants, rooted in their discovery that inulin, when used in specific concentrations and combined with such surfactants, can display outstanding thickener properties.

Accordingly, the invention provides aspects as set forth in the Summary section above. These aspects and various embodiments pertaining thereto are further elucidated herein.

The term "composition" generally refers to a thing composed of two or more components, and more specifically denotes a mixture or a blend of two or more materials, such as chemical entities (e.g., elements, molecules, substances), biological molecules (such as, without limitation, proteins, nucleic acids, lipids, and/or carbohydrates), or microbiological materials (such as, without limitation, live beneficial or probiotic bacteria, yeast, or fungi), as well as reaction products and decomposition products formed from the materials of the composition, for example formed as results of chemical processes involving the initial materials of the composition. A composition may be obtained by combining, such as admixing or blending together, e.g., mixing, dissolving, or emulsifying, the components thereof. Compositions as typically used herein may be liquid or semisolid, e.g., liquids, gels or pastes, and may include solutions or dispersions.

The adjective "cosmetic" in connection with products, uses, or methods denotes that such products, uses, or methods are designed to maintain, restore, improve, alter or enhance a subject's appearance or attractiveness. Appearance or attractiveness primarily concern aesthetic qualities such as without limitation the tone, colour, complexion, texture, smoothness, softness, or fragrance of the subject's skin, scalp, hair, eyebrows, beard, lips, teeth, or nails. For example, the primary purpose of cosmetic products, uses, or methods may be cleansing or beautifying. Cosmetic products, uses, or methods as envisaged herein address normal, natural, or physiological processes, that is routine bodily functions that do not involve disease states, and can be distinguished from therapy including curative and prophylactic treatments, the purpose of which is to restore a subject from a pathological state to its original healthy condition, or to at least alleviate the symptoms of pain and suffering caused by the pathology, or to prevent pathology in the first place. Cosmetics are used externally and do not include products that claim to have therapeutic or medicinal effects. In certain situations, cosmetics, while not preventing a disease, may protect against environmental factors affecting appearance (e.g., sunscreens). The "subject" typically and preferably denotes humans, but may also encompass reference to non-human animals, preferably warm-blooded animals, even more preferably mammals, such as, e.g., non-human primates, rodents, canines, felines, equines, ovines, porcines, etc. Examples of non-human animals in which cosmetic compositions are frequently used, for example for grooming purposes, include pet animals, such as dogs, cats, rabbits, hamsters, and the like.

The term "cosmetic composition" refers to a composition or formulation comprised of ingredients specifically selected and combined to create a product intended for cosmetic use. Such compositions are designed to achieve particular cosmetic effects, such as cleansing, moisturizing, and/or enhancing appearance.

Inulin is a naturally occurring polysaccharide belonging to the class of dietary fibers known as fructans. It is a heterogeneous collection of polymers composed of fructose units which typically have a chain-terminating glucose. The fructose units are joined by β(2→1)bonds. Inulins are almost exclusively linear with plant inulins having only a few percent branched molecules. Inulins with a terminal glucose can be denoted as GpyFn and inulins without terminal glucose as FpyFn. Inulin is produced by many types of plants, such as chicory root, Jerusalem artichoke, and agave, particularly in their roots or rhizomes, as a means of storing energy. It is industrially extracted most often from chicory root using established methods, which may typically involve the steps of harvesting and washing of the plant material, chopping the plant material into small pieces and hot water or hot ethanol extraction, filtration to separate the soluble inulin from the solid plant material, precipitation and purification of the inulin from the extract, for example by cooling the extract, and drying such as spray drying the inulin.

The present cosmetic compositions comprise at least 10% by weight of inulin based on combined weight of inulin and water in the composition. Hence, this percentage can be conveniently calculated as the weight of inulin divided by the sum of the weights of inulin and water, as expressed by the formula: weight(inulin) / (weight(inulin) + weight(water)) ≥ 0.1. By means of examples and without limitation, the present compositions may comprise at least 11%, at least 12%, at least 13%, at least 14%, at least 15%, at least 16%, at least 17%, at least 18%, or at least 19% by weight of inulin based on combined weight of inulin and water in the composition, or in further embodiments at least 20%, at least 22.5%, at least 25%, at least 27.5%, at least 30%, at least 32.5%, at least 35%, at least 37.5%, or at least 40% by weight of inulin based on combined weight of inulin and water in the composition.

In certain embodiments, the present cosmetic compositions comprise between 10% and 60%, such as between 10% and 50%, or between 10% and 40% by weight of inulin based on the combined weight of inulin and water in the composition. By means of examples and without limitation, the present compositions may comprise between 10% and 20%, between 20% and 30%, between 30% and 40%, between 40% and 50%, or between 50% and 60% by weight of inulin, based on the combined weight of inulin and water in the composition.

In certain embodiments, the present cosmetic compositions comprise at least 5% by weight of inulin based on the total weight of the composition, such as for example at least 6%, at least 7%, at least 8%, at least 9%, at least 10%, at least 11%, at least 12%, at least 13%, at least 14%, at least 15%, or at least 20%, or at least 25%, or at least 30% by weight of inulin based on the total weight of the composition. It shall be understood that where a composition comprises x % by weight of inulin based on the total weight of the composition, the percentage by weight of inulin based on the combined weight of inulin and water in the composition will be greater than x% due to the inclusion of other, non-water, components in the composition.

Inulin can be characterised *inter alia* by its degree of polymerisation (DP) which refers to the number of monomeric saccharide units (G and F in GpyFn, F in FpyFn) in the polysaccharide chain. DP determines the physicochemical properties of inulin, such as its solubility and functional behaviour in formulations. Some sources classify inulin based on DP into short-chain inulin (oligofructose, fructooligosaccharides, FOS): DP range of 2-9, optionally average DP of 4; standard inulin: DP range of 2-60, optionally average DP of 10; high-performance inulin: DP range of 10-60; and long-chain inulin: DP > 20, while other classifications are possible.

In certain embodiments, the inulin comprised in the present cosmetic compositions has a degree of polymerization (DP) between 10 and 65. In certain embodiments, the inulin comprised in the present cosmetic compositions has an average (i.e., number average) DP between 20 and 30, such as between 20 and 25, such as for example 20, 21, 22, 23, 24, or 25. This value corresponds to the total number of saccharide units (G and F units) in a given inulin sample divided by the total number of inulin molecules in said sample, without taking into account the monosaccharides glucose (G) and fructose (F) and the disaccharide sucrose (GF) which are possibly present in the sample.

The degree of polymerization (DP) and the number average DP of inulin are well-established characteristics that can be assessed using a variety of conventional techniques familiar to those skilled in the art. These may include without limitation methods such as High-Performance Liquid Chromatography (HPLC), which can separate inulin oligomers based on size, or Size-Exclusion Chromatography (SEC), also known as gel permeation chromatography, which estimates molecular size distribution by separating molecules according to their hydrodynamic volume. Mass Spectrometry (MS), particularly MALDI-TOF MS, offers another approach by providing insights into the mass distribution of oligomers, aiding in DP determination. Additionally, Enzymatic Hydrolysis, which breaks down inulin into fructose units that are subsequently quantified, can be used to infer average DP, often complementing other analytical techniques. Methodologies such as those discussed in De Leenheer L. et al., Carbohydrates as Organic Raw Materials, Ed. H. Van Bekkum et al. for CRF, Wageningen, The Netherlands, Vol. III, 67-74 (1996), may provide additional insights.

In certain embodiments, the inulin is linear inulin. A "linear polymer" refers to a polymer chain predominantly composed of monomer units linked in a continuous, unbranched sequence, i.e., for inulin a linear chain made up of β(2→1) linked fructose moieties, typically further comprising a chain-terminating glucose residue. For the purposes of defining inulin as a linear polymer, it is considered linear if at least 95% by number, such as in an increasing order of preference at least 96%, at least 97%, at least 98%, at least 99%, or even 100% by number of the polymer chains are unbranched. Any minor fraction of the polymer chains that may exhibit branching (typically involving both the β(2→1) and β(2→6) linkages between the fructose moieties) accommodates the natural variability found in plant-derived inulin, while remaining consistent with the functional properties expected of a linear polymer.

The extent of branching in inulin can be explored through various analytical techniques that offer valuable insights into its molecular structure. Techniques such as Nuclear Magnetic Resonance (NMR) Spectroscopy, particularly ¹³C NMR, are capable of identifying branching points by observing specific carbon signals linked to branching. Enzymatic hydrolysis, when paired with chromatographic methods like HPLC, can provide a way to selectively cleave linear regions, facilitating a comparative analysis of linear and branched fragments. MS, including methods such as MALDI-TOF MS, can be used to analyse mass distribution, distinguishing between linear and branched molecules based on fragmentation patterns. SEC, optionally used in conjunction with multi-angle light scattering (MALS), can offer additional insights into molecular size and structure by separating molecules according to hydrodynamic volume, which can reflect branching characteristics. Fourier Transform Infrared (FTIR) Spectroscopy may confirm the presence of branching by detecting characteristic absorption peaks associated with glycosidic linkages. These techniques, whether used individually or in combination, provide an illustration of available approaches to assessing the branching attributes of inulin.

In certain embodiments, the inulin comprised in the present cosmetic compositions has been sourced from a plant. Several plants produce and store significant quantities of inulin, particularly linear inulin, which can be isolated on industrial or commercial scale. Chicory root (*Cichorium intybus*) is one of the most significant sources, widely cultivated for its high inulin content and favourable extraction properties. Jerusalem artichoke (*Helianthus tuberosus*) tubers are another valuable source, known for their substantial inulin yield and utilisation in both food and industrial applications. Additionally, dahlia tubers (Dahlia spp.) are recognized for their inulin content, offering potential for extraction and use in various formulations. These plant sources are integral to the commercial production of inulin, providing a sustainable and efficient means of obtaining this polysaccharide for diverse applications across the food, pharmaceutical, and cosmetic industries. In particularly preferred embodiment, the inulin has been sourced from chicory root.

In certain embodiments, the inulin is natural or intact inulin. In the context of inulin, the terms "natural" or "intact" refer to inulin that is extracted from plant sources without undergoing chemical or enzymatic modifications post-harvest that alter its fundamental molecular structure or composition. "Natural" or "intact" inulin substantially maintains its original chain length, degree of polymerisation, and branching characteristics as found in the source plant, ensuring it retains the inherent physicochemical properties and functionality typical of inulin as it naturally occurs. Natural inulin is typically extracted from a source, such as a plant source, purified and isolated, without applying a treatment for reducing or increasing its (number) average DP and may typically have (number) average DP that is identical to or only slightly lower (e.g., about 1 unit lower) than the corresponding native inulin in the plant. This definition excludes any inulin that has been subjected to processes such as hydrolysis, esterification, or any other chemical treatments that change its intrinsic structure. For example, the definition excludes any inulin that has been subjected to a process, such as a chemical process, whereby the hydrogen atom of one or more of its hydroxyl groups is substituted by another group, such as a hydrophobic group (hydrophobically-modified inulin) or a charged group. The use of "natural" or "intact" inulin is thus intended to preserve the beneficial attributes associated with the inulin's native state, unaffected or only minimally affected by the processes of extraction and purification in terms of its (number) average DP, branching, and chemical structure. In particular, the inulin has not been chemically or enzymatically modified once sourced from a plant.

A "surfactant," or surface-active agent, is a compound that possesses distinct hydrophilic (water-attracting) and lipophilic (oil-attracting) domains within its molecular structure, enabling it to reduce the surface tension between two immiscible phases, such as oil and water. This dual affinity allows surfactants to aggregate at interfaces, facilitating the emulsification, dispersion, or solubilization of one phase within another. Surfactants are categorized based on the nature of their hydrophilic head group into anionic, cationic, non-ionic, and amphoteric (zwitterionic) types. They play a crucial role in various applications, including detergency, wetting, foaming, and stabilization of emulsions in industries such as cosmetics, pharmaceuticals, and food processing. Surfactants are integral to formulations where they enhance *inter alia* the spreadability, texture, and efficacy of products by modifying interfacial properties.

The Hydrophilic-Lipophilic Balance (HLB) is a numerical scale with 0 as the lower end value, used to characterize the balance between the hydrophilic (water-attracting) and lipophilic (oil-attracting) portions of a surfactant molecule. A relatively lower HLB value indicates a more lipophilic surfactant, while a relatively higher HLB value signifies a more hydrophilic surfactant.

The HLB value can be calculated based on the chemical structure of the surfactant, particularly the relative contributions of the hydrophilic and lipophilic portions of the molecule, or determined experimentally. HLB of anionic surfactants can be typically determined or calculated by Davies' method (Davies JT (1957), "A quantitative kinetic theory of emulsion type, I. Physical chemistry of the emulsifying agent", Gas/Liquid and Liquid/Liquid Interface, Proceedings of the International Congress of Surface Activity, pp. 426-38), which considers the contribution of each group within the surfactant molecule:$HLB = \sum {H}_{h , i} - \sum {H}_{l , i } + 7 ,$ where H*_{h,i}* H*_{l,i}* are the contributions of hydrophilic and hydrophobic parts, respectively. Each functional group within the molecule is assigned a specific value that reflects its hydrophilic or lipophilic nature. The sum of these values, adjusted by the base number 7, yields the HLB.

The HLB can also be determined experimentally by observing the behaviour of the surfactant in emulsification tests. Surfactants are tested in various oil-water mixtures to find the optimal HLB that stabilizes a particular emulsion. This practical approach often complements calculated values. These methods provide a systematic way to assign an HLB value to surfactants.

In certain embodiments, the present compositions may comprise: an anionic surfactant with HLB at least 20; or a non-ionic surfactant; or a mixture of an anionic surfactant with HLB at least 20 and a non-ionic surfactant .

The cosmetic compositions described herein may further comprise an amphoteric surfactant in combination with the aforementioned surfactants. Accordingly, in certain embodiments, the composition comprises a mixture of an anionic surfactant with HLB at least 20 and an amphoteric surfactant. In other embodiments, the composition comprises a mixture of a non-ionic surfactant and an amphoteric surfactant. In yet further embodiments, the composition comprises a mixture of an anionic surfactant with HLB at least 20, a non-ionic surfactant, and an amphoteric surfactant.

Accordingly, also provided is a method for thickening a cosmetic composition comprising a surfactant selected from the group consisting of an anionic surfactant with HLB at least 20, a non-ionic surfactant, and mixtures thereof, and optionally further comprising an amphoteric surfactant, the method comprising including in the composition at least 10% by weight of inulin based on the combined weight of inulin and water in the composition.

Also provided is use of inulin in an amount of at least 10% by weight based on the combined weight of inulin and water in a cosmetic composition for thickening the cosmetic composition which comprises a surfactant selected from the group consisting of an anionic surfactant with HLB at least 20, a non-ionic surfactant, and mixtures thereof, and optionally which further comprises an amphoteric surfactant.

In certain embodiments the anionic surfactant with HLB at least 20 is selected from the group consisting of sulfates, sulfosuccinates, glutamates, isethionates, olefin sulfonates, sarcosinates, taurates, glycinates, and mixtures thereof. Sulfates are characterized by a sulfate group (SO₄²⁻) attached to a hydrophobic alkyl chain. The sulfate group imparts a strong negative charge, enhancing their surfactant properties. Examples include without limitation sodium lauryl sulfate (SLS) (HLB 40), ammonium lauryl sulfate (ALS) (HLB 32-35), sodium laureth sulfate (SLES) (HLB 40), sodium-coco-sulfate (SCS) (HLB 35-40). Sulfosuccinates feature a sulfosuccinate group, which consists of a sulfonic acid moiety attached to a succinic acid backbone, linked to hydrophobic alkyl chain or chains. Examples include without limitation disodium lauryl sulfosuccinate (HLB 24-30), sodium dioctyl sulfosuccinate (HLB 25-30). Glutamates are derived from glutamic acid, comprising an amino acid moiety with a long hydrophobic chain and a carboxylate group, offering mild surfactant properties. Examples include without limitation sodium cocoyl glutamate, sodium lauroyl glutamate. Isethionates contain an isethionic acid group, which includes a sulfonate group linked to a hydroxyethyl chain, attached to a hydrophobic alkyl chain. Examples include without limitation sodium cocoyl isethionate, sodium lauroyl isethionate, sodium lauroyl methyl isethionate (HLB 25-30). The ability of inulin to thicken isethionates is exceptional. Particularly with these surfactants it forms a combination where high viscosity, great texture and stability is achieved. Isethionates in cosmetic application are very interesting as they are one of the mildest surfactants with great cleansing properties. Additionally, they give a luxury foam with pleasant after-feel. The challenge is to thicken them and ensure stability and it is very difficult to create a natural and mild rinse-off product with these surfactants. Inulin makes this possible. Olefin sulfonates are produced by sulfonating olefins, leading to a mixture of alkene sulfonates and hydroxyalkane sulfonates, offering high foaming properties. Examples include without limitation sodium C14-16 alpha olefin sulfonate (HLB 40). Sarcosinates are amino acid-based surfactants derived from sarcosine, featuring a carboxylate group and a hydrophobic alkyl chain. Examples include without limitation sodium lauroyl sarcosinate (HLB 30), sodium myristoyl sarcosinate. Taurates are taurine-based surfactants with a sulfonate group linked to an alkyl chain, known for their mildness and good foaming properties. Examples include without limitation sodium methyl cocoyl taurate (HLB 25-30), sodium lauroyl methyl taurate (HLB 25-30). Glycinates are derived from the amino acid glycine, featuring a carboxylate group and an alkyl chain, known for their mild formulation. Examples include without limitation sodium cocoyl glycinate (HLB 28-30), sodium lauroyl glycinate (HLB 28-30).

In certain embodiments, the non-ionic surfactant is selected from the group consisting of glucosides, hydroxysultaines, and mixtures thereof. Glucosides are non-ionic surfactants formed by the reaction of glucose with fatty alcohols. They consist of a sugar moiety (glucose) linked to a hydrophobic alkyl chain. The glycosidic bond connects the anomeric carbon of glucose to the alcohol group of the fatty chain. Examples include without limitation decyl glucoside, lauryl glucoside, coco glucoside, cetearyl glucoside. These surfactants are known for their mildness and biodegradability. Hydroxysultaines are zwitterionic surfactants, but they can be classified as non-ionic in formulations where their ionic character is not predominant. They contain a betaine structure with a hydroxyl group, featuring a sulfobetaine moiety linked to an alkyl chain, with a hydroxyl group providing additional hydrophilicity. Examples include without limitation lauryl hydroxysultaine and cocamidopropyl hydroxysultaine. These surfactants offer excellent foam stability and conditioning properties, often used in shampoos and body washes. These classes of non-ionic surfactants are valued for their gentle cleansing action and compatibility with various formulation components, contributing to the stability and performance of cosmetic and personal care products.

In certain embodiments, the amphoteric surfactant is selected from the group consisting of amphoacetates, betaines, and mixtures thereof. Amphoacetates are amphoteric surfactants characterized by the presence of both anionic and cationic groups within the same molecule. They typically consist of a fatty acid derivative linked to an amino acid, such as glycine or alanine, with an acetate group providing the anionic character. Examples include without limitation sodium cocoamphoacetate, disodium cocoamphodiacetate, sodium lauroamphoacetate. These surfactants are known for their mildness and conditioning properties, making them suitable for use in mild shampoos and body washes. Betaines are zwitterionic surfactants containing a quaternary ammonium group and a carboxylate group within the same molecule. The positive and negative charges are balanced, resulting in a net neutral charge. The structure typically includes a long hydrophobic alkyl chain. Examples include without limitation cocamidopropyl betaine, lauryl betaine. These surfactants are valued for their excellent foam enhancement and stabilizing properties, commonly used in personal care formulations for their gentle cleansing action. These classes of amphoteric surfactants provide versatility in formulations due to their ability to function effectively across a wide pH range, offering mildness and compatibility with other surfactant types in cosmetic and personal care products.

In certain embodiments, the composition comprises between 0.5% and 50% by weight of the recited surfactant - i.e., the surfactant selected from the group consisting of an anionic surfactant with HLB at least 20, and a non-ionic surfactant; and optionally an amphoteric surfactant - based on the total weight of the composition. In certain embodiments, the composition comprises between 0.5% and 2%, between 1% and 3%, between 2% and 5%, or between 4% and 6% by weight of the surfactant, based on the total weight of the composition. In certain embodiments, the composition comprises between 5% and 15%, between 10% and 20%, between 15% and 25%, between 20% and 30%, between 25% and 35%, between 30% and 40%, between 35% and 45%, or between 40% and 50% by weight of the surfactant, based on the total weight of the composition. In certain preferred embodiments, the composition comprises between 8% and 20% by weight of the surfactant, based on the total weight of the composition. In certain embodiments, the composition comprises between 8% and 10%, between 9% and 11%, between 10% and 13%, between 12% and 15%, between 14% and 17%, or between 15% and 20% by weight of the surfactant, based on the total weight of the composition. In certain embodiments, the composition comprises 8%, 9%, 10%, 11%, 12%, 13%, 14%, 15%, 16%, 17%, 18%, 19%, or 20% by weight of the surfactant, based on the total weight of the composition. An amount of surfactant suitable for an intended type of cosmetic composition can be adjusted as generally known. By means of an example and without limitation, a toothpaste may contain a relatively lower amount of a surfactant, such as for example between 0.5% and 2% by weight, while a shampoo or body wash can contain a relatively higher amount of a surfactant, such as for example between 8% and 20% by weight, based on the total weight of the composition. Where the composition contains more than one surfactant, the above values correspond to the total combined weight percentage of the surfactants present, based on the total weight of the composition. The total combined weight of the surfactants present is calculated as the sum of the weights of any anionic and/or non-ionic surfactants, and, if present, any amphoteric surfactant.

In certain embodiments, the composition may comprise not more than 5%, preferably no more than 4%, even more preferably no more than 3% by weight of cationic compounds based on the total weight of the composition, such as no more than 2.5%, 2%, 1.5%, 1%, 0.5%, 0.2%, 0.1%, or even 0.0% by weight. Such cationic compounds encompass any molecule or substance that carries a net positive charge due to the presence of one or more cationic groups, such as quaternary ammonium, protonated amine, or other positively charged functional groups. In cosmetic applications, cationic compounds may be employed for properties such as conditioning, antimicrobial action, film formation, and/or enhanced texture or stability of formulations. Examples of cationic compounds used in cosmetic formulations include without limitation cationic polymers, cationic starches, cationic peptides, chitosan and chitosan derivatives, and cationic dyes.

In certain preferred embodiments, the composition is substantially free of cationic surfactants. Hence, in certain embodiments, no cationic surfactant components are deliberately added when formulating the composition. Should any minor or trace amounts of a compound that could be classified as a cationic surfactant or that might exhibit cationic surfactant properties upon the intended use of the composition be present in the composition, such amounts (cumulatively in case of multiple compounds) would be limited to less than 1.0%, preferably in increasing order of preference less than 0.9%, less than 0.8%, less than 0.7%, less than 0.6%, less than 0.5%, less than 0.4%, less than 0.3%, less than 0.2%, or less than 0.1% by weight, or even 0.0% by weight, based on the total weight of the composition.

A cationic surfactant is a type of surface-active agent characterized by a hydrophilic head group that carries a positive charge, typically in the form of a quaternary ammonium ion. Main classes of cationic surfactants include quaternary ammonium compounds composed of a nitrogen atom bonded to four alkyl or aryl groups, forming a positively charged quaternary ammonium ion, such as cetyltrimethylammonium chloride (CTAC), behentrimonium chloride, benzalkonium chloride; amine salts, formed by the protonation of amine groups, resulting in cationic species, which often include long alkyl chains that enhance their surfactant properties, such as stearylamine acetate, laurylamine hydrochloride; imidazoline derivatives based on the imidazoline ring structure, which can be modified to include cationic groups, such as 2-heptadecyl-1-hydroxyethylimidazoline; and cationic polymers with repeating units that contain quaternary ammonium groups, providing a high density of positive charges along the polymer chain, such as polyquaternium-10 and polyquaternium-7.

In certain embodiments, the present compositions may comprise one or more anionic surfactant with HLB at least 20, but substantially no non-ionic, amphoteric, and cationic surfactants. In such embodiments, should any minor or trace amounts of a compound that could be classified as a non-ionic, amphoteric, or cationic surfactant or that might exhibit non-ionic, amphoteric, or cationic surfactant properties upon the intended use of the composition be present in the composition, such amounts (cumulatively in case of multiple compounds) would be limited to less than 1.0%, preferably in increasing order of preference less than 0.9%, less than 0.8%, less than 0.7%, less than 0.6%, less than 0.5%, less than 0.4%, less than 0.3%, less than 0.2%, or less than 0.1% by weight, based on the total weight of the composition.

In certain embodiments, the present compositions may comprise one or more anionic surfactant with HLB at least 20 and one or more non-ionic surfactant, but substantially no amphoteric and cationic surfactants. In such embodiments, should any minor or trace amounts of a compound that could be classified as an amphoteric or cationic surfactant or that might exhibit amphoteric or cationic surfactant properties upon the intended use of the composition be present in the composition, such amounts (cumulatively in case of multiple compounds) would be limited to less than 1.0%, preferably in increasing order of preference less than 0.9%, less than 0.8%, less than 0.7%, less than 0.6%, less than 0.5%, less than 0.4%, less than 0.3%, less than 0.2%, or less than 0.1% by weight, based on the total weight of the composition.

In certain embodiments, the present compositions may comprise one or more anionic surfactant with HLB at least 20 and one or more amphoteric surfactant, but substantially no non-ionic and cationic surfactants. In such embodiments, should any minor or trace amounts of a compound that could be classified as a non-ionic or cationic surfactant or that might exhibit non-ionic or cationic surfactant properties upon the intended use of the composition be present in the composition, such amounts (cumulatively in case of multiple compounds) would be limited to less than 1.0%, preferably in increasing order of preference less than 0.9%, less than 0.8%, less than 0.7%, less than 0.6%, less than 0.5%, less than 0.4%, less than 0.3%, less than 0.2%, or less than 0.1% by weight, based on the total weight of the composition.

In certain embodiments, the composition is a rinse-off composition rinse-off compositions are designed to be applied to the skin, hair, or scalp and then removed by rinsing with water. They are formulated to cleanse, condition, or treat the surface without leaving a residue. These compositions are often formulated to effectively remove dirt, oil, and impurities while providing a refreshing and clean feel. They typically include surfactants that facilitate cleansing and might contain conditioning agents for added benefits.

In certain embodiments, the composition is a hair treatment or hair care composition, a scalp treatment or scalp care composition, or a beard treatment or beard care composition. Hair treatment or hair care compositions aim to improve the health, appearance, and manageability of hair. They address issues such as dryness, damage, frizz, and lack of shine. These products may contain nourishing ingredients like oils, proteins, and vitamins to strengthen and repair hair. They can also include humectants and emollients to retain moisture and enhance softness. Scalp treatment or scalp care compositions focus on maintaining or restoring scalp health, addressing conditions such as dryness, flakiness, irritation, or excess oil production. They are typically formulated with soothing and moisturizing ingredients, scalp treatments may include anti-inflammatory agents, antifungal components, and exfoliants to promote a balanced scalp environment. Beard treatment or care products are designed to condition, soften, and groom facial hair while nourishing the underlying skin. These compositions often contain oils, butters, and vitamins to hydrate and tame beard hair. They may also include antimicrobial agents to support skin health beneath the beard.

In certain embodiments, the composition is a shampoo, body wash, shower gel, shower butter, soap, makeup remover, facial cleanser, scrub, or toothpaste. Shampoos are formulated to cleanse the hair and scalp by removing sebum, dirt, and product buildup. They contain surfactants for effective cleansing, typically along with conditioning agents to prevent hair from becoming dry or damaged. Shampoos may be tailored for specific hair types or concerns, such as dandruff or colour protection. Body washes are cleansers such as in the context of the present invention particularly liquid cleansers designed for use on the body during bathing or showering. They often include mild surfactants to cleanse without stripping the skin's natural oils. Body washes may be enriched with *inter alia* moisturizers, fragrances, and skin-soothing agents to enhance the bathing experience. Similar to body washes, shower gels are used for cleansing the body but typically have a thicker, gel-like consistency. They are typically formulated to lather well and rinse clean, often with added fragrances and colours for an invigorating shower experience. Shower gels may also include moisturizing ingredients to leave the skin feeling soft. Makeup removers are designed to effectively dissolve and remove makeup from the face, including stubborn or waterproof formulations. These products may come in various forms, such as oils, micellar waters, or creams, and are formulated to be gentle on the skin while efficiently breaking down makeup without causing irritation. Facial cleansers are targeted at removing dirt, oil, and impurities from the face while maintaining healthy skin balance. They are often formulated to be gentle yet effective, with variations available for different skin types, such as oily, dry, or sensitive skin. Cleansers may include hydrating and soothing ingredients to support the skin's natural barrier. Scrubs are exfoliating products designed to slough off dead skin cells, promoting smoother, brighter skin. They contain abrasive particles or chemical exfoliants that help remove dull surface cells. Scrubs are often enriched with moisturizing agents to soften the skin and prevent irritation. Toothpaste is used for oral hygiene to clean and polish teeth, remove plaque, and maintain oral health. It typically contains abrasives to aid in cleaning, fluoride for cavity prevention, and flavouring agents for a fresh taste. Some formulations are specialized for whitening, sensitivity relief, or gum health.

The disclosed cosmetic compositions may include a variety of components commonly used in the industry to enhance their effectiveness and appeal. In certain embodiments, these compositions may comprise water as a primary solvent, along with preservatives to maintain product integrity and prevent microbial growth. Stabilizers and chelating agents can be included to ensure product consistency and stability. Emulsifiers can aid to create stable mixtures of oil and water phases, while emollients provide a smooth and soft texture by forming a protective barrier on the skin or hair. Oils, such as seed oils, may be incorporated for their emollient and nourishing properties, contributing to the overall moisturizing and conditioning effects of the product. Additionally, fragrances, colorants, humectants, and further thickeners can be added to enhance the sensory attributes and performance of the composition. The preparation of these compositions involves blending the ingredients to achieve a stable and homogeneous product, followed by quality control checks and packaging under hygienic conditions. While specific embodiments have been described, it is understood that various modifications and variations can be made without departing from the teaching and scope of the invention.

The herein disclosed aspects and embodiments of the invention are further supported by the following non-limiting examples.

### EXAMPLES

One example of a rinse-off composition according to an embodiment of the invention includes 14% w/w inulin (long chain native inulin where fructose units are between 10 and 65 (average DP 20-25)) based on the combined weight of inulin and water in the composition, 30% w/w anionic surfactant sodium lauroyl glutamate (Eversoft^{™} UCS 30S), preservatives (qs), and water (qs).

This formulation, which can be optionally supplemented with further cosmetically acceptable components, gives desirable effect in terms of viscosity of the composition, evidencing the advantageous thickener action of the inulin component in the presence of an anionic surfactant.

A further example of a formulation according to an embodiment of the invention, which includes only natural ingredients is as shown in Table 1:

**Table 1.**

| **Phase** | **Ingredient** | **wt %** | **Supplier** | **INCI** |
|---|---|---|---|---|
| **A1** | Water | qs | - | Aqua |
| | Inulin | 12.5 | **Gobiotics** | Inulin |
| **A2** | Iselux^{®} LQ-CLR-SB | 35 | **Innospec** | Sodium Lauroyl Methyl Isethionate |
| | Plantacare 2000 UP | 3 | **BASF** | Decyl Glucoside |
| | Microcare^{®} NBCG | 0,6 | **Thor** | Sodium Benzoate |
| | Microcare^{®} KS | 0,1 | **Thor** | Potassium Sorbate |
| | Soy Tein NPNF | 1 | **Tri-K** | Hydrolyzed Soy Protein |
| | Inutec H25P | 2 | **Gobiotics** | Inulin |
| **B** | Jojoba Oil | 0,1 | **Olvea** | Simmondsia Chinensis Seed Oil |
| | Perfumes | 0,5 | - | - |
| | Plantacare 2000UP | 3 | **BASF** | Polyglyceryl-4 caprylate |
| | HallStar^{®} GMO | 1,5 | **Hallstar** | Glyceryl Oleate |
| **C** | Citric acid 50% | QS | **Brenntag** | Citric acid |

The weight % of inulin based on combined weight of inulin and water in the composition was 18.33%.

### Procedure:

A1 Add slowly inulin into water while mixing. Start homogenizing 10000 rpm for 10 minutes.

A2 Add slowly ingredients of A2 one by one while mixing.

B Premix ingredients of B and add the premix into A1/A2.

C Correct the pH to 5-5.5.

### Viscosity after 1 day was 16000 cPs Brookfield

pH= 5-5.5
Aspect: beige gel

Further examples of formulation according to embodiments of the invention are shown in Table 2:

**Table 2**

| **INCI** | Sodium Lauroyl Glutamate | Sodium Lauroyl Sarcosinate | Sodium Laureth Sulfate | Sodium C14-16 Olefin Sulfonate | Cocamido propyl Betaine | Cocamido propyl Hydroxy sultaine | Cetearyl Glucoside | Sodium-Coco Sulfate | Disodium Cocoamphodiacetate | Sodium Lauroyl **Methyl** Isethionate |
|---|---|---|---|---|---|---|---|---|---|---|
| Surfactant | **Eversoft UCS-30** | **Crodasini c CS30** | **Marlinat 242/70 C5** | **Ifrapan AOS 38** | **Dehyton K** | **Betadet SHR** | **Emulgade PL 68/50** | **Texapon K30 UP** | **Rewoteric AM 2 C** | **Iselux LQ-CLR-SB** |
| **Water** | qs | qs | qs | qs | qs | qs | qs | qs | qs | qs |
| **Inulin** | 14 | 14 | 14 | 14 | 14 | 16,4 | 16,8 | 14 | 17,8 | 14 |
| **Preservative** | qs | qs | qs | qs | qs | qs | qs | qs | qs | qs |
| **Surfactant** | 30 | 30 | 30 | 30 | 30 | 18 | 16 | 30 | 10 | 30 |
| **Viscosity** | 70 000 | 120 000 | 80 000 | 70 000 | 70 000 | 100 000 | 80 000 | 90 000 | 180 000 | 90 000 |
| **Aspect** | white gel | white gel | white gel | white gel | white gel | white gel | white gel | white gel | white gel | white gel |

### Procedure

Add slowly inulin into water while mixing. Start homogenizing 10000 rpm for 10 minutes.

Add the remaining ingredients one by one and mix till homogeneous.

Correct the pH to desired level.

## Claims

1. A cosmetic composition comprising:
at least 10% by weight of inulin based on combined weight of inulin and water in the composition, and
one or more surfactant selected from the group consisting of an anionic surfactant with Hydrophilic-Lipophilic Balance (HLB) at least 20, a non-ionic surfactant, and mixtures thereof.

2. The cosmetic composition according to claim 1, wherein the composition comprises between 10% and 60% by weight of inulin, or between 10% and 50% by weight of inulin, or between 10% and 40% by weight of inulin, based on the combined weight of inulin and water in the composition.

3. The cosmetic composition according to claim 1, wherein the composition comprises at least 5% by weight of inulin based on the total weight of the composition.

4. The cosmetic composition according to any one of claims 1 to 3, wherein the inulin has a degree of polymerization (DP) between 10 and 65.

5. The cosmetic composition according to any one of claims 1 to 4, wherein the inulin has an average DP between 20 and 30, optionally DP between 20 and 25.

6. The cosmetic composition according to anyone of claims 1 to 5, wherein the inulin is linear inulin.

7. The cosmetic composition according to anyone of claims 1 to 6, wherein the inulin has been sourced from a plant, optionally from chicory root.

8. The cosmetic composition according to anyone of claims 1 to 7, wherein the inulin is natural or intact inulin, optionally wherein the inulin has not been chemically or enzymatically modified once sourced from a plant.

9. The cosmetic composition according to anyone of claims 1 to 8, further comprising an amphoteric surfactant.

10. The cosmetic composition according to anyone of claims 1 to 9, wherein:
the anionic surfactant with HLB at least 20 is selected from the group consisting of sulfates, sulfosuccinates, glutamates, isethionates, olefin sulfonates, sarcosinates, taurates, glycinates, and mixtures thereof;
the non-ionic surfactant is selected from the group consisting of glucosides, hydroxysultaines, and mixtures thereof; and/or
the amphoteric surfactant is selected from the group consisting of amphoacetates, betaines, and mixtures thereof.

11. The cosmetic composition according to anyone of claims 1 to 10, wherein the composition comprises between 0.5% and 50% by weight of the surfactant, optionally between 8% and 20% by weight of the surfactant, based on the total weight of the composition.

12. The cosmetic composition according to anyone of claims 1 to 10, wherein:
the composition is a rinse-off composition;
the composition is a hair treatment or hair care composition, a scalp treatment or scalp care composition, or a beard treatment or beard care composition; or
the composition is a shampoo, body wash, shower gel, shower butter, soap, makeup remover, facial cleanser, scrub, or toothpaste.

13. A method for thickening a cosmetic composition comprising a surfactant selected from the group consisting of an anionic surfactant with HLB at least 20, a non-ionic surfactant, and mixtures thereof, and optionally further comprising an amphoteric surfactant, the method comprising including in the composition at least 10% by weight of inulin based on the combined weight of inulin and water in the composition.

14. Use of inulin in an amount of at least 10% by weight based on the combined weight of inulin and water in a cosmetic composition for thickening the cosmetic composition which comprises a surfactant selected from the group consisting of an anionic surfactant with HLB at least 20, a non-ionic surfactant, and mixtures thereof, and optionally which further comprises an amphoteric surfactant,.

15. The method according to claim 13 or the use according to claim 14, wherein:
- the composition comprises between 10% and 60% by weight of inulin, or between 10% and 50% by weight of inulin, or between 10% and 40% by weight of inulin, based on the combined weight of inulin and water in the composition, optionally wherein the composition comprises at least 5% by weight of inulin based on the total weight of the composition;
- the inulin has a degree of polymerization (DP) between 10 and 65;
- the inulin has an average DP between 20 and 30, optionally DP between 20 and 25;
- the inulin is linear inulin;
- the inulin has been sourced from a plant, optionally from chicory root;
- the inulin is natural or intact inulin, optionally wherein the inulin has not been chemically or enzymatically modified once sourced from a plant;
- the anionic surfactant with HLB at least 20 is selected from the group consisting of sulfates, sulfosuccinates, glutamates, isethionates, olefin sulfonates, sarcosinates, taurates, glycinates, and mixtures thereof;
- the non-ionic surfactant is selected from the group consisting of glucosides, hydroxysultaines, and mixtures thereof;
- the optional amphoteric surfactant is selected from the group consisting of amphoacetates, betaines, and mixtures thereof;
- the composition comprises between 0.5% and 50% by weight of the surfactant, optionally between 8% and 20% by weight of the surfactant, based on the total weight of the composition;
- the composition is a rinse-off composition;
- the composition is a hair treatment or hair care composition, a scalp treatment or scalp care composition, or a beard treatment or beard care composition; and/or
- the composition is a shampoo, body wash, shower gel, shower butter, soap, makeup remover, facial cleanser, scrub, or toothpaste.
